# EUROPEAN PATENT SPECIFICATION

(11) **EP 2 551 324 B1**
(45) Date of publication and mention of the grant of the patent: **01.01.2014**
(21) Application number: 11006300.5
(22) Date of filing: 29.07.2011
(51) Int. Cl.: C09K 5/14, B29C 55/00, H01L 23/373

(54) **Use of an anisotropic fluoropolymer for the conduction of heat**
Verwendung eines anisotropen Fluorpolymers zur Wärmeleitung
Utilisation d'un fluoropolymère anisotrope pour la conduction de la chaleur

(43) Date of publication of application: 30.01.2013
(73) Proprietor: W.L.GORE & ASSOCIATES GMBH, 85639 Putzbrunn (DE)
(72) Inventor: Bürger, Wolfgang, 84424 Burgrain (DE); Wendlandt, Michael, 85521 Ottobrunn (DE); Schlichting, Leonard, 83626 Oberlaindern (DE)
(74) Representative: Kador & Partner

(56) References cited:
- US-A- 3 953 566
- US-A- 3 962 153
- US-A- 5 738 936
- US-A- 5 945 217
- US-A1- 2004 180 209

## Description

The present invention relates to the use of an anisotropic fluoropolymer with an enhanced intrinsic thermal conductivity in at least one direction as a heat conducting material, to a thermally conductive article comprising said anisotropic fluoropolymer and to a process for the production of said anisotropic fluoropolymer.

Fluoropolymers such as polytetrafluoroethylene (PTFE) are known to have a low intrinsic thermal conductivity which for dense, isotropic PTFE is less than 0.35 W/mK within a wide range of temperatures from -140°C to at least 232°C, see e.g. Price, D. M. & Jarratt, M. (2002), "Thermal conductivity of PTFE and PTFE composites", Thermochimica Acta 392-393, p. 231-236 or Blumm, J.; Lindemann, A.; Meyer, M. & Strasser, C. (2010), "Characterization of PTFE Using Advanced Thermal Analysis Techniques", International Journal of Thermophysics 31, 1919-1927.

It is known that the thermal conductivity of expanded porous PTFE is generally even lower due to the presence of air, typically only one tenth to about one half of the value for the dense material as a function of the porosity. These materials have therefore found application as thermal insulators (see e.g. US 3,953,566, column 5, line 64 to column 6, line 2).

For applications as e.g. for the heat transfer from integrated circuits ("IC") it is desired to make use of the advantageous properties of fluoropolymers, but, at the same time, thermal conductivity is required. It is known to make fluoropolymers such as PTFE thermally conductive by the incorporation of thermally conductive particles, for example metal particles, oxides or nitrides, and PCM or elastomers. Such thermally conductive PTFE composites are disclosed e.g. in US 5,945,217 and US 5,738,936.

However, the use of such filled fluoropolymers has several drawbacks, in particular there is usually a loss of the outstanding properties of PTFE like stability against harsh chemical environments or a change of dielectric properties.

It is therefore one of the objects of the present invention to avoid the drawbacks of these known systems and to provide a fluoropolymer for heat conducting applications which combines the unique features of fluoropolymers, such as stability against harsh chemical environments, high temperatures or UV-light, low dielectric constant, electric insulating properties, flexibility, repellency to water and other liquids, with a thermal conductivity which is high enough as to allow the production of thermally conductive articles.

The present invention is based on the surprising finding that a tremendous increase in the intrinsic thermal conductivity of fluoropolymers can be obtained by orienting the fluoropolymer in at least one direction, and that the increase in thermal conductivity is obtained in the direction of orientation while the thermal conductivity in the other direction(s) slightly decreases or remains the same, thus creating a thermally anisotropic fluoropolymer.

The present invention therefore provides the use of an anisotropic fluoropolymer having a different intrinsic thermal conductivity in at least two directions as a heat conducting material in a thermally conductive article.

The fact that the anisotropic fluoropolymers show a different intrinsic thermal conductivity in at least two directions means that they show a comparatively high intrinsic thermal conductivity in one direction and a comparatively low intrinsic thermal conductivity in another direction, which is usually perpendicular to the direction of high intrinsic thermal conductivity. This is caused by an orientation of the polymer chains in the fluoropolymer which makes the fluoropolymer anisotropic, with the direction of high thermal conductivity being parallel to the direction of the chain orientation.

The described use solves the objects of the present invention, in particular allows for the use of fluoropolymers as heat conducting material in thermally conductive articles without the need of adding thermally conductive fillers. The increased ability to conduct heat is intrinsic to the pure fluoropolymer and, hence, the advantageous properties of fluoropolymers such as mentioned above are maintained.

Furthermore, as no additives are necessary to make the fluoropolymer thermally conductive, undesired water adsorbance due to the presence of such additives is avoided. This is especially important for the application in electrical components.

Still further, as the thermal conductivity is low in one direction and high in another direction, heat can be conducted very precisely in those direction(s) with high thermal conductivity, whereas in the other direction(s) the fluoropolymer is basically insulating.

Finally, the production of thermally anisotropic fluoropolymers is comparatively simple and, hence, cost-effective, compared to known systems, and procedures known in the art for orienting of fluoropolymers can be used with small modifications.

The thermally anisotropic fluoropolymer may be partially fluorinated or fully fluorinated, i.e. perfluorinated.

In one embodiment, the fluoropolymer comprises, or consists of, polytetrafluoroethylene (PTFE), a modified PTFE, a fluorothermoplastic or a fluoroelastomer or any combination of these materials. The term "modified PTFE" as used herein is intended to denote a type of tetrafluoroethylene copolymer in which in addition to tetrafluoroethylene monomer units further perfluorinated, fluorinated or non-fluorinated co-monomer units are present, for example in a range of from 0.005 to 15 mol%.

In a second embodiment, the substrate consists of polytetrafluoroethylene (PTFE), a modified PTFE, a fluorothermoplastic or a fluoroelastomer or any combination of these materials.

In a further embodiment, the fluoropolymer comprises, or consists of, PTFE and/or a modified PTFE, and in still a further embodiment, the fluoropolymer comprises, or consists of, PTFE.

The intrinsic conductivity of the anisotropic fluoropolymer is the thermal conductivity which is present within the pure fluoropolymer, i.e. without the addition of any further compound or filler.

In one embodiment, the intrinsic thermal conductivity of the fluoropolymer is 0.5 W/mK or more, in a further embodiment is 0.7 W/mK or more, in still a further embodiment is 1 W/mK or more, in a still further embodiment is 5 W/mK or more, and in still a further embodiment is 8 W/mK or more, in the direction of maximum intrinsic thermal conductivity.

All indications of thermal conductivities and diffusivities as well as any ratios thereof as contained herein relate to a measurement temperature of 40 °C, unless otherwise indicated.

Usually, the intrinsic thermal conductivity in the direction of maximum intrinsic thermal conductivity does not exceed 40 W/mK, due to the limited expansion/drawability of fluoropolymers.

Of course, intrinsic thermal conductivities as high as possible are desired, however, for certain applications a lower conductivity may be acceptable, for example, where the amounts of heat to be transferred is smaller.

The anisotropic fluoropolymer in the use of the present invention has different intrinsic thermal conductivities in at least two directions, with the anisotropy ratio of the intrinsic thermal conductivity being defined as the ratio of the intrinsic thermal conductivity in the direction of maximum intrinsic thermal conductivity divided by the intrinsic thermal conductivity in the direction of minimum intrinsic thermal conductivity.

In one embodiment, the fluoropolymer has an anisotropy ratio of the intrinsic thermal conductivity of more than 5, in another embodiment of 10 or more, in still a further embodiment of 15 or more, in still a further embodiment of 20 or more, in still a further embodiment of 25 or more, in still a further embodiment of 30 or more, and in still a further embodiment of 40 or more.

In principle, an anisotropy ratio of the intrinsic thermal conductivity as high as possible is desirable. However, in practice, the ratio will usually not exceed 100.

To make full use of the benefits of the present invention, in one embodiment the anisotropic fluoropolymer does not comprise a thermally conductive filler, or, in a further embodiment, any filler or further compound at all.

As a thermally conductive filler such compounds are defined which have an intrinsic thermal conductivity of 1 W/mK or more in at least one direction.

However, it is, in principle, not excluded that in order to still increase the thermal conductivity of the anisotropic polymer to add thermally conductive fillers.

Usually, the anisotropic fluoropolymer is in the form of a fiber or a sheet, this being due to the fact that the enhanced intrinsic thermal conductivity in at least one direction is obtained by an orientation of the polymer chains.

The term "fiber" is intended to denote all articles with an extension in one dimension being large compared to the extension in the other two dimensions, e.g. articles usually denoted as fibers, filaments or threads. The term "sheet" is intended to denote all articles with an extension in two dimension being large compared to the extension in the other, remaining dimension, e.g. articles usually denoted as sheets, films, membranes or tapes.

Fibers may, for example, be manufactured from sheets by slitting the sheet parallel to the fiber axis.

In the embodiment where the fluoropolymer is in the form of fibers, which are oriented in the direction of the fiber axis, the axial intrinsic thermal conductivity κ_{α} corresponds to the maximum intrinsic thermal conductivity whereas the radial intrinsic thermal conductivity κᵣ corresponds to the direction of minimum intrinsic thermal conductivity. Thus, the anisotropy ratio of the intrinsic thermal conductivity is κₐ / κᵣ.

Furthermore, in this embodiment the axial thermal diffusivity is in, one embodiment, more than 0.2 mm²/s, in another embodiment more than 1 mm²/s, in still another embodiment more than 5 mm²/s, and in still another embodiment more than 9 mm²/s.

Usually, the axial thermal diffusivity does not extend 22 mm²/s.

In a further embodiment the anisotropic fluoropolymer is in the form of a sheet. The sheet may be oriented in one direction parallel to the plane of the sheet (monoaxial orientation) or in two directions perpendicular to each other parallel to the plane of the sheet (biaxial orientation).

The intrinsic thermal conductivity will be increased in the direction(s) of orientation, so that the sheet will show an increased intrinsic thermal conductivity along one direction within the plane (monoaxial orientation), or along all directions within the plane (biaxial orientation).

The anisotropic fluoropolymer may be a dense material, i.e. a non-porous material, or may be porous.

The term "porous" as used herein refers to a material which has voids throughout the internal structure which may form an interconnected continuous air path from one surface to the other.

The anisotropic fluoropolymer may also be microporous. This means that the voids are very small and are usually referred to as "microscopic".

A typical pore size of the voids in a microporous fluoropolymer is in the range of 0.01 to 15 micrometer as determined in the mean flow pore size measurement.

A microporous fluoropolymer, for example, is expanded PTFE (ePTFE, EPTFE).

The microstructure of a porous fluoropolymer can include nodes and fibrils, only fibrils, only fibril strands or bundles, or stretched nodes interconnected by fibrils.

In one embodiment in the use of the present invention the thermally conductive article has a thermal conductivity of 0.5 W/mK or more, in a further embodiment 0.7 W/mK or more and still in a further embodiment 1 W/mK and still a further embodiment 5 W/mK or more, or in a further embodiment 8 W/mK or more, in the direction of maximum thermal conductivity of the article.

In a further embodiment, the anisotropic fluoropolymer has not been modified so as to make it electrically conducting, i.e. is still an electrical insulator. Thus, in this embodiment the surface resistivity of the anisotropic fluoropolymer is 10¹⁰ Ohm/square or more at 20°C and 42% relative humidity.

The intrinsic thermal conductivity of the anisotropic fluoropolymer changes less than 35% in the range of 40 °C to 180 °C.

In embodiments of the use according to the invention, the thermally conductive article be also one according to any of the below described embodiments.

The present invention also provides a thermally conductive article for the conduction of heat from a heat source to a heat sink comprising an isotropic fluoropolymer having a different intrinsic thermal conductivity in at least two directions wherein the fluoropolymer is arranged in the article in such a way that during use of the article heat is conducted in the fluoropolymer from the heat source to the heat sink in the direction of maximal intrinsic thermal conductivity, and wherein the thermally conductive article has a thermal conductivity of 0.5 W/mk or more in the direction of maximum thermal conductivity of the article.

The terms "heat source" and "heat sink" are intended to denote any item which gives off heat and receives heat, respectively.

Embodiments of the thermally conductive article may comprise the anisotropic fluoropolymer in any of the embodiments for the anisotropic fluoropolymer described above.

The thermally conductive article of the invention in addition to the anisotropic fluoropolymer may comprise further components, such as a matrix compound, serving to keep the anisotropic fluoropolymer in the described arrangement within the article.

Usually, such an article comprising further components in addition to the anisotropic fluoropolymer is denoted as "composite".

In one embodiment such matrix compounds may also be fluoropolymers so as to not impair the advantages of the anisotropic fluoropolymer for the thermally conductive article.

The thermally conductive article provides all the benefits as mentioned for the use according to the invention above. In particular, it is possible to make a thermally conductive article which is stable against harsh chemical environments, high temperatures, or UV-light, has a high dielectric strength, electrical insulating properties, flexibility, repellency to water and other liquids, good dielectric properties, shows a good ageing behavior and a stable conductivity over a broad temperature range.

Still further, the article allows for the conduction of heat very precisely in one or two dimensions, depending on the orientation of the anisotropic fluoropolymer.

In one embodiment, the thermally conductive article has a thermal conductivity of 0.7 W/mK, in a further embodiment of 1 W/mK or more, in still a further embodiment of 5 W/mK or more, or in still a further embodiment of 8 W/mK or more, in the direction of maximum thermal conductivity of the article.

The overall thermal conductivity of the article in its different directions is determined by all its components, i.e. the anisotropic fluoropolymer and, where applicable, further components such as matrix compounds.

The thermal conductivity in the thermally conductive article according to one embodiment is anisotropic, with the anisotropy ratio being defined as the ratio between the direction of maximum thermal conductivity and the thermal conductivity in the direction of minimum thermal conductivity.

In one embodiment, the thermally conductive article has an anisotropy ratio of the intrinsic thermal conductivity of more than 2, in another embodiment more than 5, in still another embodiment of 10 or more, in still a further embodiment of 15 or more, in still a further embodiment of 20 or more, in still a further embodiment of 25 or more, in still a further embodiment of 30 or more, and in still a further embodiment of 50 or more.

To make full benefit of the advantages offered by the use of the anisotropic fluoropolymer in one embodiment the article does not comprise other thermally conductive materials than the fluoropolymer.

Thermally conductive material is defined to be a material which has a thermal conductivity in the direction of maximum thermal conductivity of 1 W/mK or more.

In still a further embodiment, the thermally conductive article is an electrical insulator so that it has a surface resistivity of 10¹⁰ Ohm/square or more at 20°C and 42% relative humidity.

The thermally conductive article may be in any form or shape known for such articles in the art, in particular the article may be a woven mat, a laminate, a fiber resin composite, a thermal interface composite, an electrically insulating heat spreader or a heat pipe.

For the production of the thermally anisotropic fluoropolymer the polymer chains in the fluoropolymer must be oriented. This can be done by known procedures in the art, however, care has to be taken that after orientation the improved heat conductivity in the direction of orientation is not lost by further treatment.

In particular, it has been discovered that, for example, where stretching/expanding is used as a means for orientation of the fluoropolymer, a sintering or annealing step after the stretching/expansion step(s) is detrimental to the thermal conductivity in the direction of orientation.

Thus, the present invention also relates to a process for the production of an anisotropic fluoropolymer having a different intrinsic thermal conductivity in at least two directions wherein a fluoropolymer precursor is oriented in at least one direction and wherein the fluoropolymer after orientation is not subjected to a sintering or annealing treatment, and wherein orientation is performed by stretching the fluoropolymer precursor at a temperature from 280 to 420°C.

In one embodiment of the process, orientation is performed by stretching the fluoropolymer precursor with a stretching rate of 5 %/s or more, in a further embodiment of 10 %/s or more, in a still a further embodiment of 50 %/s or more, and in still a further embodiment of 70%/s or more.

The latter two embodiments especially apply for the case of the fluoropolymer being in the form of fibers.

In a further embodiment of the process, orientation is performed by stretching the fluoropolymer precursor with a stretching rate of 1000 %/s or less, in a further embodiment of 500 %/s or less, and in still a further embodiment of 100 %/s or less.

In another embodiment of the process, orientation is performed by stretching the fluoropolymer precursor at a temperature of from 330 to 400 °C, and in another embodiment at temperature of from 340 to 380 °C.

Furthermore, the process according to the invention is for the manufacture of the anisotropic fluoropolymer in any of the above described embodiments.

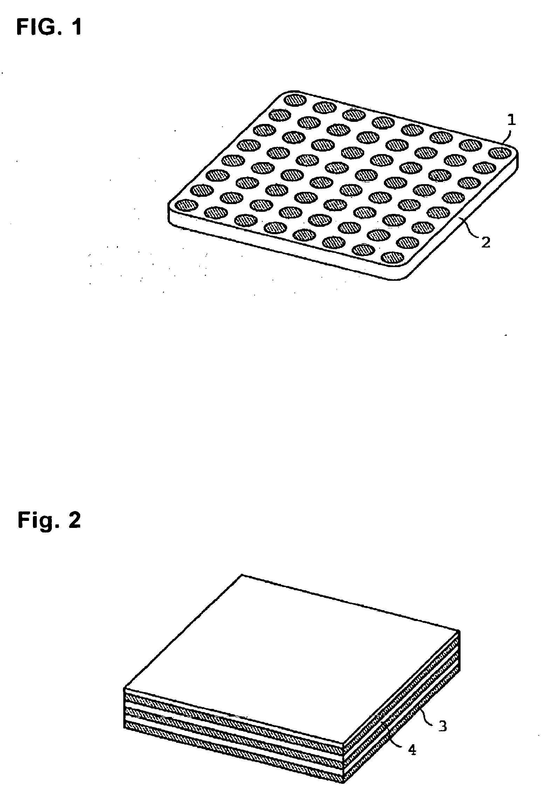

In Fig. 1, a fiber resin composite is shown in which axially thermally conductive PTFE fiber or fiber bundles (1) are arranged by a compliant matrix (2), with the direction of heat conduction being parallel to the fiber bundles.

In Fig. 2, a woven fiber mat with in-plane thermal conductivity (3) and through-plain thermal insulation (4) is shown.

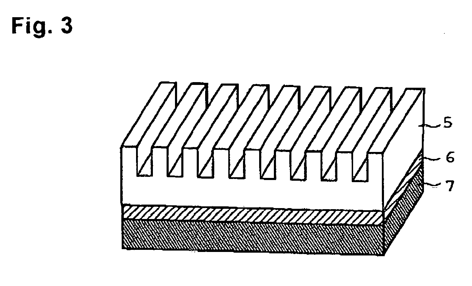

In Fig. 3, heat transfer from a processor (7) to a heat sink (5) is shown with a thermal interface composite (6) which may e.g. be that shown in Fig. 1.

The present invention will be further illustrated by the examples described below.

### Methods and Examples

Fibers were prepared from sheetlike materials, e.g. tapes by slitting into narrow strips and strechting them as described in example 1. It is assumed that the measured thermal properties of fibers also apply to samples with other aspect ratios, e.g. highly stretched sheets or membranes, if they were strechted under the same conditions.

In addition, a membrane has been prepared from the precursor tape as described in Example 2.

### a) Measurement of thermal properties

Fiber samples for thermal analysis were prepared from continous fibers as described in Example 1. Bundles of parallel fibers of 8 cm length were aligned within a shrink hose made of PVF. The shrink hose was shrinked at 180°C for 15 minutes in order to produce cylinders comprising a bundle of parallel fibers held together by the shrink hose. The diameter of the cylindrical fiber bundles, which can be described as fiber-air composites, was between 15mm and 20mm. Thermal properties were measured according to ISO 22007-2 using a Hot Disk TPS 2500S thermal constants analyser.

The thermal properties of the single fiber (see Table 2 and 3) were calculated from the apparent thermal properties of the fiber bundle, which can be described as a fiber-air composite. From the apparent density of the fiber bundle and the apparent density of the fibers, the actual volume fraction of the fibers in the fiber-air composite was determined. Using this and a thermal conductivity of air of 0.0262 W K⁻¹ m⁻¹, the actual thermal properties of the fibers were calculated as described in Fujishiro, H.; Ikebe, M.; Kashima, T. & Yamanaka, A. (1997), "Thermal Conductivity and Diffusivity of High-Strength Polymer Fibers", Japanese Journal of Applied Physics 36 (Part 1, No. 9A), 5633-5637, equations 1 and 2.

The anisotropy ratio, which is defined as the ratio of the axial to the radial thermal conductivity of a fiber, has been estimated using the literature value of ca. 0.3 W K⁻¹ m⁻¹, as reported for PTFE in Blumm, J.; Lindemann, A.; Meyer, M. & Strasser, C. (2010), 'Characterization of PTFE Using Advanced Thermal Analysis Techniques', International Journal of Thermophysics 31, 1919-1927.

The determination of the radial thermal properties of the single fibers from the measured apparent values of the fiber bundle suffers from the unknown thermal contact resistance between the bundled fibers, however, there is strong experimental evidence that the reported literature values fit the actual radial thermal properties of the fibers within an acceptable range.

An average relative error of about 10% was estimated for thermal properties from data scatter.

### b) Measurement of Mechanical Properties

Fiber samples for tensile tesing were prepared from continous fibers as described in Example 1.

The apparent density of the fibers is the mass per unit volume including voids inherent in the material as tested. It was measured by a liquid displacement method using water containing 0.05 vol% TRITON X-100 wetting agent to lower the surface tension of the water.

The specific density is the mass per unit lenth including voids inherent in the material as tested. It was measured according to ISO 2060. Values are reported in dtex.

The specific tensile strength was measured acccording to ISO 2062. The specific tensile modulus was determined from the steepest slope within the elastic regime of the force-deflection curve measured according to ISO 2062.

Shrinkage was measured from free end shrinking of 1 m long fibers at 250°C for 15 minutes according to ASTM D4974-01. Values are determined from the ratio of the change in length by shrinking to the initial unshrinked length.

### c) Measurement of Electrical Properties

Surface resistivity was measured according ASTM D 257 set up between two parallel electrodes with a square configuration with a Keithley Model 617 Electrometer. The temperature was set to 21 °C with relative humidity of 42 %.

### d) Measurement of Water Uptake

Fiber samples for water uptake measurements were prepared from continous fibers as described in example 1.

The water uptake was measured according to ISO 4611. The mass of the conditioned specimen conditioned at 23°C and 50% relative humidity for 86 h was determined by weighing. After the conditioning step, the specimen were subjected to a constant climate at 40°C and 90% relative humidity for 24 h, and weighted again. Water uptake was determined by the relative difference in mass per surface area of samples equilibrated at the two conditions described above.

### EXAMPLE 1

Following the procedures disclosed in U.S. Pat. Nos. 3,953,566, 3,962,153, and 4,064,214 a precursor fiber was prepared in the following manner:

A fine powder PTFE resin was mixed with mineral spirit (22.6 wt% Isopar K™) to form a paste and extruded through a die to form a wet tape of 0.980 mm thickness. Subsequently, the wet tape was rolled down, stretched at a ratio of 1 to 0.75 and than dried at 185° C to remove the mineral spirit. The dry tape had a final thickness of 0.415 mm and was slit to 4.31 mm widths by passing it between a set of gapped blades to serve as precursor fibers.

The precursor fibers were stretched over hot plates at 350°C to 370°C, at a total stretch ratio as shown in Table 1 and a stretch rate exceeding 75 %/s to form a fiber.

After stretching, the fibers were not subjected to any further treatment at elevated temperature.

The precursor fiber (sample ID F0) and stretched specimen (sample ID F1-F3) were measured to determine mechanical properties and thermal properties by the methods described herein. The results are shown in Tables 1-3.

### EXAMPLE 2

Following the procedures disclosed in U.S. Pat. Nos. 3,953,566, 3,962,153, and 4,064,214 a precursor fiber was prepared in the following manner:

A fine powder PTFE resin was mixed with mineral spirit (20.9 wt% Isopar K™) to form a paste and extruded through a die to form a wet tape of 0.980 mm thickness. Subsequently, the wet tape was rolled down, stretched at a ratio of 1 to 0.71 and than dried at 185° C to remove the mineral spirit. The dry tape had a final thickness of 0.352 mm.

The dry tape was stretched over hot plates at 300°C, at a total stretch ratio as shown in Table 1 and a stretch rate exceeding 10%/s. After stretching, the tape was not subjected to any further treatment at elevated temperature.

The tape (sample ID M1) was measured to determine mechanical properties, thermal properties, and electrical properties by the methods described herein. The results are shown in Tables 1-2 and 4.

**Table 1: Mechanical Properties**

| Sample ID | Total stretch ratio λ | Apparent density [g/cm³] | Specific density ρₗᵢₙ [dtex] | Axial specific tensile strength σₜ [cN/dtex] | Axial specific tensile modulus Eₜ [cN/dtex] | Axial tensile strength [GPa] | Axial tensile modul us [GPa] | Shrinkage [%] |
|---|---|---|---|---|---|---|---|---|
| F0 | 1 | 1,49 | 21127 | 0.14 | 0.9 | 0.02 | 0.13 | 2.2 |
| F1 | 40 | 1.25 | 1317 | 4.97 | 185.9 | 0.62 | 23.24 | 4.6 |
| F2 | 80 | 1.72 | 661 | 5.41 | 299.1 | 0.93 | 51.45 | 8.2 |
| F3 | 140 | 1.68 | 380 | 5.37 | 410.5 | 0.90 | 69.96 | 6.9 |
| M1 | 40 | 1.21 | n.a. | n.a. | n.a. | 0.09 | 2.59 | 26. |

**Table 2: Thermal Properties at 40°C**

| Sample ID | Axial thermal conductivity κₐ [W K⁻¹ m⁻¹] | Axial thermal diffusivity αₐ [mm²/s] | Estimated Anisotropy ratio κₐ/κᵣ |
|---|---|---|---|
| F0 | 0.9 | 0.6 | 3 |
| F1 | 7.8 | 5.9 | 26 |
| F2 | 10.5 | 7.3 | 35 |
| F3 | 14.3 | 9.5 | 48 |
| M1 | 4.6 | 3.6 | 15 |

**Table 3: Thermal Properties**

| Sample ID | Tem pera ture [°C] | Axial thermal conductivity Kₐ [W K⁻¹ m⁻¹] | Axial thermal diffusivity αₐ [W K⁻¹ m⁻¹] | Anisotropy ratio κₐ/κᵣ |
|---|---|---|---|---|
| F3 | 0 | 17 | 12.8 | 57 |
| F3 | 60 | 13.5 | 8.9 | 45 |
| F3 | 120 | 12.9 | 8.0 | 43 |
| F3 | 180 | 10.2 | 5.9 | 34 |

**Table 4: Electrical Properties and Water Uptake**

| Sample ID | Electrical Surface Resistivity [Ohm/square] | Water uptake |
|---|---|---|
| F0 | n.a. | <0.05% |
| F1 | n.a. | <0.05% |
| F2 | n.a. | <0.05% |
| F3 | n.a. | <0.05% |
| M1 | > 10¹¹ | n.a. |

## Claims

1. Use of an anisotropic fluoropolymer having a different intrinsic thermal conductivity in at least two directions as a heat conducting material in a thermally conductive article.

2. Use according to claim 1, wherein the fluoropolymer is polytetrafluoroethylene (PTFE), a modified PTFE, a fluorothermoplastic, a fluoroelastomer or any combination thereof.

3. Use according to any of the preceding claims, wherein the intrinsic thermal conductivity of the fluoropolymer is 0.5 W/mK or more in the direction of maximum intrinsic thermal conductivity.

4. Use according to any of the preceding claims, wherein the fluoropolymer has an anisotropy ratio of the intrinsic thermal conductivity of 5 or more.

5. Use according to any of the preceding claims, wherein the fluoropolymer does not comprise a thermally conductive filler.

6. Use according to any of the preceding claims, wherein the fluoropolymer is in the form of a fiber or a sheet.

7. A thermally conductive article for the conduction of heat from a heat source to a heat sink comprising an anisotropic fluoropolymer having a different intrinsic thermal conductivity in at least two directions wherein the fluoropolymer is arranged in the article in such a way that in use of the article heat is conducted in the fluoropolymer from the heat source to the heat sink in the direction of maximum thermal conductivity, and wherein the thermally conductive article has a thermal conductivity of 0.5 W/mK or more in the direction of maximum thermal conductivity of the article.

8. Thermally conductive article according to 7 wherein the thermally conductive article has a thermal conductivity anisotropy ratio of 2 or more.

9. Thermally conductive article according to any of claims 7 or 8 wherein the article does not comprise a thermally conductive material other than the fluoropolymer.

10. Thermally conductive article according to any of claims 7 to 9 wherein the thermally conductive article has an electrical resistivity of 10¹⁰ Ohm/sq. or more at 20 °C.

11. Thermally conductive article according to any of claims 7 to 10 wherein the article is a woven mat, a laminate, a fiber-resin composite, a thermal interface composite, an electrically insulating heat spreader, or a heat pipe.

12. A process for the production of an anisotropic fluoropolymer having a different intrinsic thermal conductivity in at least two directions wherein a fluoropolymer precursor is oriented in at least one direction and wherein the fluoropolymer after orientation is not subjected to a sintering or annealing treatment, and wherein orientation is performed by stretching the fluoropolymer precursor at a temperature from 280 to 420 °C.

13. Process according to claim 12 wherein orientation is performed by stretching the fluoropolymer precursor with a stretching rate of 50%/s or more.

14. Process according to any of claims 12 to 13 wherein the thermal conductivity of the oriented fluoropolymer is 0.5 W/mK or more in the direction of maximum thermal conductivity.

15. Process according to any of claims 12 to 14 wherein the fluoropolymer has a thermal conductivity anisotropy ratio of 5 or more.

## Patentansprüche

1. Verwendung eines anisotropen Fluorpolymers mit einer unterschiedlichen intrinsischen Wärmeleitfähigkeit in mindestens zwei Richtungen als ein wärmeleitendes Material in einem wärmeleitfähigen Artikel.

2. Verwendung gemäß Anspruch 1, wobei das Fluorpolymer Polytetrafluorethylen (PTFE), ein modifiziertes PTFE, ein Fluorelastomer oder irgendeine Kombination davon ist.

3. Verwendung gemäß einem der vorhergehenden Ansprüche, wobei die intrinsische Wärmeleitfähigkeit des Fluorpolymers 0,5 W/mK oder mehr in Richtung der maximalen intrinsischen Wärmeleitfähigkeit beträgt.

4. Verwendung gemäß einem der vorhergehenden Ansprüche, wobei das Fluorpolymer ein Anisotropie-Verhältnis der intrinsischen Wärmeleitfähigkeit von 5 oder mehr aufweist.

5. Verwendung gemäß einem der vorhergehenden Ansprüche, wobei das Fluorpolymer keinen wärmeleitfähigen Füllstoff umfasst.

6. Verwendung gemäß einem der vorhergehenden Ansprüche, wobei das Fluorpolymer in Form einer Faser oder einer Folie vorliegt.

7. Wärmeleitfähiger Artikel für die Wärmeleitung von einer Wärmequelle zu einer Wärmesenke, umfassend ein anisotropes Fluorpolymer mit einer unterschiedlichen intrinsischen Wärmeleitfähigkeit in mindestens zwei Richtungen, wobei das Fluorpolymer in dem Artikel derart angeordnet ist, dass bei Verwendung des Artikels Wärme in dem Fluorpolymer von der Wärmequelle zur Wärmesenke in Richtung der maximalen Wärmeleitfähigkeit geleitet wird und wobei der wärmeleitfähige Artikel eine Wärmeleitfähigkeit von 0,5 W/mK oder mehr in Richtung der maximalen Wärmeleitfähigkeit des Artikels aufweist.

8. Wärmeleitfähiger Artikel gemäß Anspruch 7, wobei der wärmeleitfähige Artikel ein Wärmeleitfähigkeits-Anisotropie-Verhältnis von 2 oder mehr aufweist.

9. Wärmeleitfähiger Artikel gemäß einem der Ansprüche 7 oder 8, wobei der Artikel kein anderes wärmeleitfähiges Material als das Fluorpolymer umfasst.

10. Wärmeleitfähiger Artikel gemäß einem der Ansprüche 7 bis 9, wobei der wärmeleitfähige Artikel einen spezifischen elektrischen Widerstand von 10¹⁰ Ohm/sq. oder mehr bei 20 °C aufweist.

11. Wärmeleitfähiger Artikel gemäß einem der Ansprüche 7 bis 10, wobei der Artikel eine gewebte Matte, ein Laminat, ein Faser-Harz-Verbundmaterial einen thermisches Interface-Verbundmaterial, ein elektrisch isolierender Wärmespreizer oder ein Wärmerohr ist.

12. Verfahren zur Herstellung eines anisotropen Fluorpolymers mit einer unterschiedlichen intrinsischen Wärmeleitfähigkeit in mindestens zwei Richtungen, wobei ein Fluorpolymer-Vorläufer in mindestens eine Richtung ausgerichtet wird und wobei das Fluorpolymer nach der Ausrichtung keinem Sintern oder einer Glühbehandlung unterzogen wird und wobei die Ausrichtung durch Strecken des Fluorpolymer-Vorläufers bei einer Temperatur von 280 bis 420 °C durchgeführt wird.

13. Verfahren gemäß Anspruch 12, wobei die Ausrichtung durch Strecken des Fluorpolymer-Vorläufers mit einer Streckungsrate von 50%/s oder mehr durchgeführt wird.

14. Verfahren gemäß einem der Ansprüche 12 bis 13, wobei die Wärmeleit'fähigkeit des ausgerichteten Fluorpolymers 0,5 W/mK oder mehr in Richtung der maximalen Wärmeleitfähigkeit beträgt.

15. Verfahren gemäß einem der Ansprüche 12 bis 14, wobei das Fluorpolymer Wärmeleitfähigkeits-Anisotropie-Verhältnis von 5 oder mehr aufweist.

## Revendications

1. Utilisation d'un fluoropolymère anisotrope présentant une conductivité thermique intrinsèque différente dans au moins deux directions en tant que matériau conducteur de chaleur dans un article thermiquement conducteur.

2. Utilisation selon la revendication 1, dans lequel le fluoropolymère est le polytétrafluoroéthylène (PTFE), un PTFE modifié, un fluorothermoplastique, un fluoroélastomère ou une combinaison quelconque de ceux-ci.

3. Utilisation selon l'une quelconque des revendications précédentes, dans laquelle la conductivité thermique intrinsèque du fluoropolymère est supérieure ou égale à 0,5 W/mK dans le sens de la conductivité thermique intrinsèque maximale.

4. Utilisation selon l'une quelconque des revendications précédentes, dans laquelle le fluoropolymère présente un rapport d'anisotropie de la conductivité thermique intrinsèque supérieur ou égal à 5.

5. Utilisation selon l'une quelconque des revendications précédentes, dans laquelle le fluoropolymère ne comprend pas une charge thermiquement conductrice.

6. Utilisation selon l'une quelconque des revendications précédentes, dans laquelle le fluoropolymère se présente sous la forme d'une fibre ou une feuille.

7. Article thermiquement conducteur pour la conduction de chaleur depuis une source de chaleur vers un dissipateur de chaleur comprenant un fluoropolymère anisotrope présentant une conductivité thermique intrinsèque différente dans au moins deux directions dans lesquelles le fluoropolymère est agencé dans l'article de telle manière que lors de l'utilisation de l'article, la chaleur est dirigée dans le fluoropolymère de la source de chaleur vers le dissipateur de chaleur dans le sens de conductivité thermique maximale et où l'article thermiquement conducteur présente une conductivité thermique supérieure ou égale à 0,5 W/mK dans le sens de conductivité thermique maximale de l'article.

8. Article thermiquement conducteur selon la revendication 7, dans lequel l'article thermiquement conducteur présente un rapport d'anisotropie de conductivité thermique supérieur ou égal à 2.

9. Article thermiquement conducteur selon l'une quelconque des revendications 7 ou 8, dans lequel l'article ne comprend pas de matériau thermiquement conducteur autre que le fluoropolymère.

10. Article thermiquement conducteur selon l'une quelconque des revendications 7 à 9, dans lequel l'article thermiquement conducteur présente une résistivité électrique supérieure ou égale à 10¹⁰ Ohm/sq. à 20 °C.

11. Article thermiquement conducteur selon l'une quelconque des revendications 7 à 10, dans lequel l'article est un tapis tissé, un stratifié, un composite fibre-résine, un composite d'interface thermique, un dissipateur thermique électriquement isolant, ou un caloduc.

12. Procédé de production d'un fluoropolymère anisotrope présentant une conductivité thermique intrinsèque différente dans au moins deux sens dans lesquelles un précurseur de fluoropolymère est orienté dans au moins un sens et dans lequel le fluoropolymère après l'orientation n'est pas soumis à un traitement de frittage ou de recuit et dans lequel l'orientation est effectuée en étirant le précurseur de fluoropolymère à une température allant de 280 à 420 °C.

13. Procédé selon la revendication 12 dans lequel l'orientation est effectuée en étirant le précurseur de fluoropolymère à un taux d'étirage supérieur ou égal à 50 %/s.

14. Procédé selon l'une quelconque des revendications 12 à 13 dans lequel la conductivité thermique du fluoropolymère orienté est supérieure ou égale à 0,5 W/mK dans le sens de conductivité thermique maximale.

15. Procédé selon l'une quelconque des revendications 12 à 14 dans lequel le fluoropolymère présente un rapport d'anisotropie de conductivité thermique supérieur ou égal à 5.
